(19) Europäisches Patentamt
European Patent Office
Office européen des brevets

(11) Veröffentlichungsnummer: **0 276 766 B1**

(12) # EUROPÄISCHE PATENTSCHRIFT

(45) Veröffentlichungstag der Patentschrift: **09.09.92**

(51) Int. Cl.[5]: **C07C 33/34**, C07C 29/36, C07C 33/20

(21) Anmeldenummer: **88100887.4**

(22) Anmeldetag: **22.01.88**

(54) **Verfahren zur Herstellung von Beta-Aryl-alkoholen.**

(30) Priorität: **29.01.87 DE 3702621**

(43) Veröffentlichungstag der Anmeldung:
**03.08.88 Patentblatt 88/31**

(45) Bekanntmachung des Hinweises auf die Patenterteilung:
**09.09.92 Patentblatt 92/37**

(84) Benannte Vertragsstaaten:
**CH DE ES FR GB IT LI NL**

(56) Entgegenhaltungen:
**US-A- 4 250 200**
**US-A- 4 265 818**

**BULLETIN OF THE CHEMICAL SOCIETY OF JAPAN, Band 53, Nr. 2, Februar 1980, Seiten 458-463, The Chemical Society of Japan, Tokyo, JP; M. INOUE et al.: "Friedel-crafts reaction of aromatics with epoxides. Stereochemistry and selectivities"**

(73) Patentinhaber: **BASF Aktiengesellschaft**
**Carl-Bosch-Strasse 38**
**W-6700 Ludwigshafen(DE)**

(72) Erfinder: **Eckhardt, Heinz, Dr.**
**Ruedigerstrasse 7**
**W-6700 Ludwigshafen(DE)**
Erfinder: **Eggersdorfer, Manfred, Dr.**
**Hannongstrasse 18**
**W-6710 Frankenthal(DE)**

EP 0 276 766 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von $\beta$-Arylalkoholen der allgemeinen Formel I

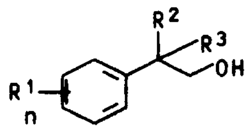

I,

in der die Reste $R^1$, $R^2$ und $R^3$ gleich oder verschieden sind und für Wasserstoff, $C_1$-$C_{20}$-Alkyl, $C_5$-$C_7$-Cycloalkyl, Aryl, $C_7$-$C_{12}$-Aralkyl oder -Alkaryl stehen, n die Zahl 0, 1, 2, 3 oder 4 bedeutet oder in der zwei benachbarte Reste $R^1$ zu einem 5- oder 6-gliedrigen Ring verknüpft sind, durch Umsetzung eines Aromaten der Formel II

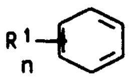

II

mit einem Alkylenoxid der Formel III

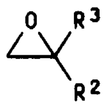

III,

wobei $R^1$, $R^2$ und $R^3$ die obengenannte Bedeutung haben, in Gegenwart von Friedel-Crafts-Katalysatoren.

Die Umsetzung von aromatischen Verbindungen mit Alkylenoxiden in Gegenwart von Friedel-Crafts-Katalysatoren ist gut bekannt. Dabei ist es wichtig, die Reaktionstemperatur unter 10°C zu halten; insbesondere bei aromatischen Verbindungen mit tert. Alkylresten, wie alkylsubstituiertem Indan oder 1,2,3,4-Tetrahydronaphthalin sind Temperaturen unter -10°C erforderlich. Im Falle der Verwendung halogenierter Kohlenwasserstoffe als Lösungsmittel werden die Ausgangsstoffe sogar bei Temperaturen unter -18°C umgesetzt (US-PS 3 532 719). Diese extremen Bedingungen sind technisch jedoch nur mit hohem Aufwand zu realisieren, zumal die Umsetzung in der Regel stark exotherm verläuft.

Aus der Literatur sind verschiedene Vorschläge bekannt, um dieses Problem zu lösen. In der EP 89207 wird. z.B. vorgeschlagen, die Umsetzung bei -15°C im Vakuum durchzuführen, so daß das Lösungsmittel siedet und so die Reaktionswärme abführt. Zur Kondensation des Lösungsmittels ist jedoch eine Kühlung auf sehr tiefe Temperaturen mit Trockeneis nötig, um den Lösungsmittelverlust gering zu halten; das Verfahren ist daher sehr kostenaufwendig. Nach US-PS 4 162 256 wird eine Erhöhung der Reaktionstemperatur auf -20 bis -5°C durch Zugabe eines aliphatischen Kohlenwasserstoffs als Lösungsmittel erreicht. Die hier angegebenen Beispiele zeigen deutlich die Temperaturabhängigkeit der Reaktion. Bei -10°C beträgt die Ausbeute bezogen auf Propylenoxid 42 bis 44 % (Bsp. 1, 2 und 4), bei -5°C jedoch nur noch 27 % (Bsp. 3). Der erzielte Umsatz des Ausgangsstoffes I ist sehr niedrig und erfordert eine hohe Rückführungsquote. Die Problematik der Reaktionsführung ist deshalb auch hierdurch nicht zufriedenstellend gelöst.

Der Erfindung lag daher die Aufgabe zugrunde, die beschriebene Friedel-Crafts-Alkylierung unter Vermeidung der oben geschilderten Nachteile durchzuführen.

Es wurde nun ein Verfahren zur Herstellung der eingangs definierten $\beta$-Arylalkohole der allgemeinen Formel I durch Umsetzung eines Aromaten der Formel II

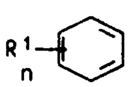

II

mit einem Alkylenoxid der Formel III

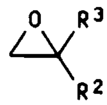

III

in Gegenwart von Friedel-Crafts-Katalysatoren gefunden, das dadurch gekennzeichnet ist, daß man die Umsetzung in Gegenwart eines Titan(IV)alkoholates bei Temperaturen von -20 ° C bis +40 ° C in Gegenwart oder in Abwesenheit eines Lösungsmittels durchführt.

Bei den erfindungsgemäß verwendeten Titan(IV)alkoholaten, die auch als Titansäureester oder Orthotitanate bezeichnet werden, handelt es sich um Verbindungen der allgemeinen Formel IV

$Ti(OR^4)_4$ IV ,

wobei $R^4$ einen inerten organischen Rest, z.B. einen Alkyl-, Cycloalkyl-, Aralkyl- oder Arylrest darstellt. Alkylreste sind z.B. verzweigte oder unverzweigte $C_1$-$C_{12}$-, insbesondere $C_1$-$C_8$-Alkylreste wie Methyl, Ethyl, Propyl, Isopropyl, Butyl, Isobutyl, Pentyl, Hexyl oder Dodecyl. Cycloalkylreste sind z.B. Cyclopentyl- oder Cyclohexyl-, Aryl- oder Aralkylreste z.B. der Phenyl oder Benzylrest. Aufgrund der leichten Verfügbarkeit sind Tetraalkyltitanate, insbesondere solche, in denen $R^4$ eine $C_1$-$C_6$-Alkylgruppe bedeutet, besonders bevorzugt.

Beispielsweise seien folgende Titanate genannt:
Tetramethyl-, Tetraethyl-, Tetrapropyl-, Tetraisopropyl-, Tetrabutyl-titanat.

Bereits geringe Mengen an Titan(IV)alkoholaten führen zu einer deutlichen Verfahrensverbesserung. So können vorteilhaft Mengen von 0,002 bis 0,1, insbesondere 0,005 bis 0,05 mol pro Mol Ausgangsstoff II verwendet werden. Größere Mengen sind möglich, bringen aber im allgemeinen keine weiteren Vorteile.

Die Umsetzung kann in Abwesenheit oder vorteilhaft in Gegenwart von Lösungs- oder Verdünnungsmitteln durchgeführt werden, wobei die an sich für Friedel-Crafts-Reaktionen üblichen Lösungsmittel verwendet werden können. Besonders vorteilhaft können als Lösungsmittel halogenierte aliphatische und aromatische Kohlenwasserstoffe und/oder überschüssiger Ausgangsstoff II verwendet werden. Die Lösungsmittel können in reiner Form oder als Mischungen eingesetzt werden.

Beispiele für aliphatische und cycloaliphatische Kohlenwasserstoffe sind Pentan, Hexan, Petrolether, Cyclohexan, 2,2,4-Trimethylpentan oder Octan. Beispiele für halogenierte aliphatische Kohlenwasserstoffe sind halogenierte Alkane, Alkene oder Cycloalkane wie Dichlormethan, Chloroform, Tetrachlormethan, 1,2-Dichlorethan, 1,1-Dichlorethan, 1,1,2-Trichlorethan, 1,1,2,2-Tetrachlorethan, 1,2-Dichlorpropan, 1,3-Dichlorpropan, 1,1,2-Trichlor-1,2,2-Trifluorethan, Trichlorethen, Tetrachlorethen. Aromatische Halogenkohlenwasserstoffe sind z.B. Chlorbenzol oder Dichlorbenzol.

Die Reaktionstemperatur kann zwischen -20 und 40 ° C gewählt werden. Die optimale Temperatur ist abhängig von den verwendeten Ausgangsstoffen II und III und vom Losungsmittel und muß im speziellen Fall bestimmt werden. Dabei sind in der Regel bei Verwendung von Kohlenwasserstoffen und/oder überschüssigen Ausgangsstoff II als Lösungsmittel Temperaturen von -10 ° C bis +40 ° C insbesondere 0 bis 30 ° C zweckmäßig. Der entsprechende Temperaturbereich bei Verwendung von Halogenkohlenwasserstoffen liegt vorteilhaft zwischen -20 und +20 ° C, insbesondere bei -10 bis + 15 ° C. Die Umsetzungen können auch bei den bislang üblichen tiefen Temperaturen durchgeführt werden, jedoch bringt der Zusatz an Titan(IV)alkoholat dann keine wesentlichen Vorteile.

Als Friedel-Crafts-Katalysatoren können die an sich üblichen Verbindungen, insbesondere Titantetrachlorid und besonders bevorzugt Aluminiumchlorid verwendet werden, wobei Mengen von 0,5 bis 4, bevorzugt 0,8 bis 1,5 mol Katalysator je Mol Ausgangsstoff II zweckmäßig sind.

In den Ausgangsstoffen II kann der Rest $R^1$ für Wasserstoff, $C_1$-$C_{20}$-, insbesondere $C_1$-$C_8$-, vorzugsweise $C_5$-$C_6$-Cycloalkyl, Aryl wie Phenyl oder $C_7$-$C_{12}$-Aralkyl oder -Alkenyl, z.B. Phenylalkyl wie Benzyl oder Phenylethyl oder für Tolyl stehen. Im Fall n größer 1 können die Reste $R^1$ gleich oder verschieden sein. Die genannten Reste können auch unter den Reaktionsbedingungen inerte Substituenten wie $C_1$-$C_4$-Alkyl oder aromatisch gebundenes Halogen tragen. Beispielsweise seien folgende Reste $R^1$ genannt: Methyl, Ethyl, Propyl Isopropyl, Butyl, Isobutyl, tert.-Butyl, Cyclopentyl, Cyclohexyl, Phenyl, Tolyl, Benzyl oder Phenylethyl.

Zwei benachbarte Reste $R^1$ können auch miteinander zu einem 5- oder 6-gliedrigen Ring, der gegebenenfalls Substituenten, insbesondere $C_1$-$C_4$-Alkylgruppen, z.B. Methylgruppen, tragen kann, verknüpft sein.

Bevorzugte Ausgangsstoffe II sind: Benzol, Toluol, o,p,m-Xylol, Ethylbenzol, Isopropylbenzol, tert.-Butylbenzol, Indan oder 1,2,3,4-Tetrahydronaphthalin (Tetralin®) sowie deren alkylsubstituierte Derivate wie 1,1,2,3,3-Pentamethylindan, 1-Ethyl-1,3,3-trimethylindan oder 1,1,4,4-Tetramethyl-1,2,3,4-tetrahyronaphthalin.

In den Alkylenoxiden III können die Reste $R^2$ und $R^3$ die für $R^1$ genannte Bedeutung haben, d.h. für Wasserstoff, $C_1$-$C_{20}$-Alkyl, $C_5$-$C_8$-Cycloalkyl, Aryl, $C_7$-$C_{12}$-Aralkyl oder -Alkaryl stehen. Bevorzugt sind Alkylenoxide, in denen $R^2$ und $R^3$ Wasserstoff oder eine niedermolekulare Alkylgruppe mit 1 bis 8, insbesondere 1 bis 4 Kohlenstoffatomen bedeuten. Beispielsweise kommen folgende Verbindungen in Betracht: Ethylenoxid, 1-Methyl-,1,1-Dimethyl-,1-Ethyl-, 1-Propyl-, 1-Isopropyl-, 1-Butyl-, 1-sek.-Butyl-, 1-tert. -Butyl-, 1-Pentyl- und 1-Hexyl-ethylenoxid. Besonders bevorzugte Ausgangsstoffe III sind Ethylenoxid, Propylenoxid, Butylenoxid-(1,2) oder 1,1-Dimethylethylenoxid.

Je Mol Ausgangsstoff II können vorteilhaft 0,5 bis 4, vorzugsweise 1 bis 2 mol Akylenoxid III eingesetzt werden. Die Umsetzung kann vorteilhaft in Gegenwart von 0,1 bis 3, vorzugsweise 0,3 bis 2 l Lösungsmittel je Mol II vorgenommen werden.

Die Durchführung der Umsetzung kann vorteilhaft in der Weise erfolgen, daß man in einem Rührbehälter den Ausgangsstoff II mit dem Titanalkoholat IV und dem Aluminiumchlorid zusammen mit einem Teil des Lösungsmittels vorlegt. Im Verlauf von 1 bis 10 h wird eine Mischung aus Lösungsmittel und Alkylenodix III bei der gewünschten Reaktionstmperatur zudosiert, wobei die Menge des Lösungsmittels in der Zulauflösung vorzugsweise so bemessen werden sollte, daß die Konzentration des Alkylenoxids III maximal 60 % beträgt. Es ist jedoch auch möglich, die gesamte Lösungsmittelmenge vorzulegen. Die Zulauflösung kann sowohl über der Flüssigkeitsoberfläche als auch darunter zudosiert werden; im letzten Fall ist es zweckmäßig, gleichzeitig einen Stickstoffstrom durch die Lösung zu leiten. Nach beendeter Reaktionszeit kann das Reaktionsgemisch nach an sich bekannten Methoden z.B. Zugabe von Wasser, Trennen der Phasen und Destillation aufgearbeitet werden.

Nach dem erfindungsgemäßen Verfahren ist es überraschend möglich, die Umsetzung der aromatischen Verbindungen II mit Alkylenoxiden III in Gegenwart von Friedel-Crafts-Katalysatoren wie $AlCl_3$ mit guten Resultaten bei höheren Temperaturen als gemäß dem Stand der Technik durchzuführen.

Als Lösungsmittel können vorzugsweise auch halogenierte Kohlenwasserstoffe eingesetzt werden, von denen bekannt war, daß zur Erzielung guter Ausbeuten und hoher Umsätze bevorzugt tiefe Temperaturen gewählt werden müssen. Ein weiterer Vorteil des erfindungsgemäßen Verfahrens ist, daß die Umsetzung in üblichen Reaktionsgefäßen durchgeführt werden kann und insbesondere keine speziellen Kühlaggregate notwendig sind. Infolge der höheren Reaktionstemperatur verläuft die Umsetzung schneller, die Reaktionswärme läßt sich infolge geringerer Viskosität leichter abführen und die Reaktionszeit kann gering gehalten werden, so daß Zersetzungs-reaktionen und die Bildung hochsiedender Nebenprodukte unterbunden werden.

Die nach dem erfindungsgemäßen Verfahren vorteilhaft herstellbaren $\beta$-Arylalkohole werden entweder selbst als Riechstoff verwendet, z.B. Phenylethanol, Tolylethanol oder dienen als Zwischenprodukte zur Herstellung von Riechstoffen wie z.B. 2'-(1,1,2,3,3-Pentamethylindanyl-5)-propanol sowie zur Herstellung von Pflanzenschutzmitteln oder Pharmazeutika.

Beispiele 1 bis 3

Herstellung von 2'-(1,1,2,3,3-Pentamethylindanyl-5)-propanol in Dichlormethan

Bei einer Temperatur von -10 °C wurden 63 g (0,3 mol) 1,1,2,3,3-Pentamethylindan zusammen mit 44 g (0,33 mol) $AlCl_3$ und in Tabelle 1 angegebenen Menge Titantetrabutylat in 50 ml Dichlormethan vorgelegt. Unter Rühren und Einleiten von Stickstoff wurden innerhalb von 1 h bei der in Tabelle 1 angegebenen Temperatur 19,3 g (0,33 mol) Propylenoxid, gelöst in 50 ml des Lösungsmittels, zugegeben. Nach Hydrolyse des Reaktionsgemisches wurde die Ausbeute gaschromatographisch bestimmt.

Tabelle 1:

| Beispiel | $Ti(OC_4H_9)_4$ g | mol | Temperatur °C | Ausbeute % a) | b) |
|---|---|---|---|---|---|
| 1 | 6 | $1,76 \cdot 10^{-2}$ | 0 | 37 | 92 |
| 2 | 6 | $1,76 \cdot 10^{-2}$ | +10 | 29 | 92 |
| 2a | - | - | +10 | 20 | 43 |
| 3 | 3 | $0,88 \cdot 10^{-2}$ | -10 | 35 | 95 |

b) bezogen auf Propylenoxid
b) bezogen auf umgesetztes Pentamethylindan

Beispiel 4 bis 15

Herstellung von 2'-(1,1,2,3,3-Pentamethylindanyl-5)-propanol in verschiedenen Lösungsmitteln

Man verfuhr wie im Beispiel 1, verwendete jedoch nur 1,5 g ($0,44 \bullet 10^{-2}$ mol) Titan-tetrabutylat und anstelle des Dichlormethans die in Tabelle 2 angegebenen Lösungsmittel. Die Reaktionstemperatur betrug -5 bis 0 ° C.

Tabelle 2

| Beispiel | Lösungsmittel | Ausbeute a) | b) |
|---|---|---|---|
| 4 | Chlorbenzol | 38 % | 93 % |
| 5 | Trichlorethen | 32 % | 95 % |
| 6 | 1,2-Dichlorethan | 34 % | 80 % |
| 7 | Tetrachlorethen | 25 % | 89 % |
| 8 | 1,1,2-Trichlorethan | 46 % | 90 % |
| 9 | 1,2-Dichlorpropan | 26 % | 90 % |
| 10 | 1,1,2-Trichlor-1,2,2-trifluorethan | 22 % | 91 % |
| 11 | o-Dichlorbenzol | 45 % | 96 % |
| 12 | 1,1,2,2-Tetrachlorethan | 41 % | 96 % |
| 13 | 1,2-Dichlorethen | 32 % | 91 % |
| 14 | 50 % 1,2-Dichlorethan<br>50 % Tetrachlorethen | 39 % | 92 % |
| 15 | 50 % 1,2-Dichlorpropan<br>50 % Tetrachlorethen | 44 % | 91 % |

a) bezogen auf Propylenoxid
b) bezogen auf umgesetztes Pentamethylindan

Beispiel 16

Bei -10 ° C wurden 100 g (0,5 mol) 1,1,2,3,3-Pentamethylindan zusammen 88 g (0,66 mol) $AlCl_3$ und 3 g ($0,88 \bullet 10^{-2}$ mol) Titantetrabutylat in 100 ml Dichlormethan vorgelegt. Unter Rühren und Einleiten von Stickstoff wurden bei -5 ° C bis 0 ° C innerhalb von 8 h 38,6 g (0,66 mol) Propylenoxid, gelöst in 100 ml Dichlormethan zugegeben. Man erhält nach Destillation 46 g unumgesetztes 1,1,2,3,3-Pentamethylindan (54 % Umsatz) und 64,4 g 2′-(1,1,2,3,3-Pentamethylindanyl-5)-propanol vom Kp. 140 bis 145 ° C/l mbar, entsprechend einer Ausbeute von 91 %, bezogen auf umgesetztes 1,1,2,3,3-Pentamethylindan.

Beispiel 17

Bei -10 ° C wurden 94 g (0,50 mol) 1,1,2,3,3-Pentamethylindan zusammen mit 80 g (0,6 mol) $AlCl_3$ in 100 ml Tetrachlorethen und 100 ml 1,2-Dichlorpropan und 2,5 g ($0,73 \bullet 10^{-2}$ mol) Titan-tetrabutylat vorgelegt. Unter Rühren und Einleiten von Stickstoff wurden bei -5 ° C bis 0 ° C innerhalb von 2 h 35 g (0,6 mol) Propylenoxid, gelöst in 100 ml Tetrachlorethen und 100 ml 1,2-Dichlorpropan zugegeben. Man erhält nach Destillation 31 g 1,1,2,3,3-Pentamethylindan (67 % Umsatz) und 71 g 2′-(1,1,2,3,3-Pentamethylindanyl-5)-propanol, entsprechend einer Ausbeute von 86 %, bezogen auf umgesetztes 1,1,2,3,3-Pentamethylindan und 48 % bezogen auf eingesetztes Propylenoxid.

Beispiel 18

Herstellung von 2′-(1,1,2,3,3-Pentamethylindanyl-5)-propanol in Abwesenheit bzw. Gegenwart von Titantetraisopropylat

In einem 250 ml Rührbehälter wurden vorgelegt: 44 g (0,33 mol) $AlCl_3$, 63 g (0,3 mol) 1,1,2,3,3-Pentamethylindan und 50 ml Cyclohexan. Unter Rühren und Einleiten von Stickstoff wurden bei 25 bis 30 ° C innerhalb von 1 h eine Mischung aus 19,3 g (0,33 mol) Propylenoxid und 50 ml Cyclohexan zugegeben. Nach insgesamt 2 h Reaktionszeit wurde hydrolysiert und die Ausbeute gaschromatographisch bestimmt. Der Umsatz an 1,1,2,3,3-Pentamethylindan betrug 50 %, die Ausbeute an 2′-(1,1,2,3,3-Pentamethylindanyl-5)-propanol, bezogen auf umgesetztes 1,1,2,3,3-Pentamethylindan 5 %. Es wurden hauptsächlich höhersiedende Nebenprodukte erhalten. Führte man die Umsetzung dagegen in Gegenwart von 25 g ($8,8x10^{-2}$ mol) Titantetraisopropylat, so stieg die Ausbeute, bezogen auf umgesetztes 1,1,2,3,3-Pentamethylindan, auf 80 % an.

Beispiele 19 und 20

Herstellung bon 2-Tolylethanol

400 g Dichlorethan und 110 g (1,2 mol) Toluol wurden zusammen mit 63 g (0,47 mol) $AlCl_3$ in Gegenwart von 3 g ($10^{-2}$ mol) Titantetraisopropylat bzw. in Abwesenheit von Titanalkoholat vorgelegt und innerhalb von 1,5 h bei der in Tabelle 3 angegebenen Temperatur mit 44 g (1 mol) Ethylenoxid versetzt. Nach Hydrolyse wurde das Reaktionsgemisch eingeengt und gaschromatographisch analysiert. Die Ausbeuten an 2-Tolylethanol, das als Isomerengemisch anfällt, bezogen auf umgesetztes Toluol, ist der nachfolgenden Tabelle zu entnehmen:

Tabelle 3

| Beispiel | $Ti(Oi-C_3H_7)_4$ [mol] | Temperatur [ ° C] | Ausbeute [%] |
|---|---|---|---|
| 19 | 0,01 | 0 | 96 |
| 19a | - | 0 | 89 |
| 20 | 0,01 | + 10 | 56 |
| 20a | - | + 10 | 42 |

## Patentansprüche

1. Verfahren zur Herstellung von β-Arylalkoholen der allgemeinen Formel I

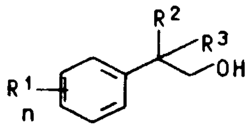

I,

in der die Reste $R^1$, $R^2$ und $R^3$ gleich oder verschieden sind und für Wasserstoff, $C_1$-$C_{20}$-Alkyl, $C_5$-$C_7$-Cycloalkyl, Aryl, $C_7$-$C_{12}$-Aralkyl oder -Alkaryl stehen, n die Zahl 0, 1, 2, 3 oder 4 bedeutet oder in der zwei benachbarte Reste $R^1$ zu einem 5- oder 6-gliedrigen Ring verknüpft sind, durch Umsetzung eines Aromaten der Formel II

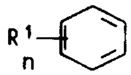

II

mit einem Alkylenoxid der Formel III

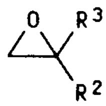

III,

wobei $R^1$, $R^2$ und $R^3$ die oben genannte Bedeutung haben, in Gegenwart von Friedel-Crafts-Katalysatoren, dadurch gekennzeichnet, daß man die Umsetzung in Gegenwart eines Titan(IV)alkoholates bei Temperaturen von -20 °C bis +40 °C in Gegenwart oder in Abwesenheit eines Lösungsmittels durchführt.

**2.** Verfahren nach Anspruch 1, dadurch gekennzeichnet, daß man ein Titan(IV)alkoholat der Formel IV

$Ti(OR^4)_4$ IV ,

in der $R^4$ eine $C_1$-$C_6$-Alkylgruppe bedeutet, verwendet.

**3.** Verfahren nach den Ansprüchen 1 und 2, dadurch gekennzeichnet, daß man 0,002 bis 0,1 mol Titan(IV)alkoholat je Mol Ausgangsstoff II verwendet.

**4.** Verfahren nach Anspruch 1, dadurch gekennzeichnet, daß man die Umsetzung in halogenierten Kohlenwasserstoffen als Lösungsmittel bei Temperaturen von -20 bis +20 °C durchführt.

**5.** Verfahren nach Anspruch 1, dadurch gekennzeichnet, daß man die Umsetzung in Kohlenwasserstoffen oder in Gegenwart von überschüssigem Ausgangsstoff II als Lösungsmittel bei Temperaturen von -10 bis +40 °C durchführt.

**6.** Verfahren nach Anspruch 1, dadurch gekennzeichnet, daß man als Friedel-Crafts-Katalysator Aluminiumchlorid verwendet.

**7.** Verfahren nach Anspruch 1, dadurch gekennzeichnet, daß man als Aromat II Indan, 1,2,3,4-Tetrahydronaphthalin oder deren alkylsubstituierte Derivate verwendet.

## Claims

**1.** A process for preparing a $\beta$-aryl alcohol of the general formula I

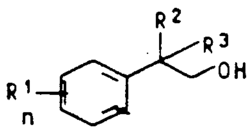

I

where $R_1$, $R^2$ and $R^3$ are identical or different and each is hydrogen, $C_1$-$C_{20}$-alkyl, $C_5$-$C_7$-cycloalkyl, aryl, $C_7$-$C_{12}$-aralkyl or $C_7$-$C_{12}$-alkaryl, n is 0, 1, 2, 3 or 4, or where two adjacent radicals $R^1$ are linked to form a 5- or 6-membered ring, by reacting an aromatic of the formula II

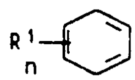

II

with an alkylene oxide of the formula III

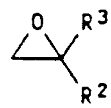

III

where $R^1$, $R^2$ and $R^3$ have the abovementioned meanings, in the presence of a Friedel-Crafts catalyst, which comprises carrying out the reaction in the presence of a titanium(IV) alcoholate at from -20 to +40 °C in the presence or absence of a solvent.

**2.** A process as claimed in claim 1, wherein a titanium(IV) alcoholate of the formula IV

$Ti(OR^4)_4$ IV

where $R^4$ is $C_1$-$C_6$-alkyl, is used.

**3.** A process as claimed in claim 1 or 2, wherein from 0.002 to 0.1 mole of titanium(IV) alcoholate is used per mole of starting material II.

**4.** A process as claimed in claim 1, wherein the reaction is carried out in a halogenated hydrocarbon as solvent at from -20 to +20 °C.

**5.** A process as claimed in claim 1, wherein the reaction is carried out in a hydrocarbon or in the presence of excess starting material II as solvent from -10 to +40 °C.

**6.** A process as claimed in claim 1, wherein the Friedel-Crafts catalyst used is aluminum chloride.

**7.** A process as claimed in claim 1, wherein the aromatic II used is indan, 1,2,3,4-tetrahydronaphthalene or an alkyl-substituted derivative thereof.

**Revendications**

**1.** Procédé de préparation d'alcools $\beta$-aryliques de formule générale I

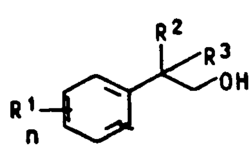

I,

dans laquelle les restes $R^1$, $R^2$ et $R^3$ sont identiques ou différents et sont mis pour des atomes

d'hydrogène ou pour des radicaux alkyle en $C_1$-$C_{20}$, cycloalkyle en $C_5$-$C_7$, aryle, aralkyle ou alcaryle en $C_7$-$C_{12}$, n est mis pour le nombre 0, 1, 2, 3 ou 4, ou dans laquelle deux restes $R^1$ voisins sont reliés en un noyau penta- ou hexagonal, par réaction d'un carbure aromatique de formule II

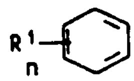

II

avec un oxyde d'alkylène de formule III

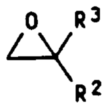

III.

$R^1$, $R^2$ et $R^3$ ayant les significations données ci-dessus, en présence de catalyseurs de Friedel-Crafts, caractérisé en ce qu'on conduit la réaction en présence d'un alcoolate de titane (IV) à des températures de -20 ° C à + 40 ° C, en présence ou en l'absence d'un solvant.

**2.** Procédé selon la revendication 1, caractérisé en ce qu'on utilise un alcoolate de titane (IV) de formule IV

$Ti(OR^4)_4$ IV

dans laquelle $R^4$ représente un groupement alkyle en $C_1$-$C_6$.

**3.** Procédé selon la revendication 1 ou 2, caractérisé en ce qu'on utilise de 0,002 à 0,1 mole d'alcoolate de titane (IV) par mole de substance de départ II.

**4.** Procédé selon la revendication 1, caractérisé en ce qu'on conduit la réaction dans des hydrocarbures halogénés servant de solvant, à des températures de -20 à + 20 ° C.

**5.** Procédé selon la revendication 1, caractérisé en ce qu'on conduit la réaction dans des hydrocarbures ou en présence de substance de départ II en excès servant de solvant, à des températures de -10 à + 40 ° C.

**6.** Procédé selon la revendication 1, caractérisé en ce qu'on utilise du chlorure d'aluminium comme catalyseur de Friedel-Crafts.

**7.** Procédé selon la revendication 1, caractérisé en ce qu'on utilise, comme carbure aromatique II, de l'indane, du 1,2,3,4-tétrahydronaphtalène ou leurs dérivés alkyl-substitués.